Europäisches Patentamt
European Patent Office
Office européen des brevets

(19)

(11) Publication number: **0 136 693**
**A2**

(12) **EUROPEAN PATENT APPLICATION**

(21) Application number: 84111752.6

(22) Date of filing: 02.10.84

(51) Int. Cl.4: **C 07 H 19/06**

(30) Priority: 03.10.83 JP 185316/83
25.05.84 JP 106990/84

(43) Date of publication of application:
10.04.85 Bulletin 85/15

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Furukawa, Yoshiyasu
7-12, Nakasujiyamate 2-chome
Takarazuka Hyogo 665(JP)

(74) Representative: von Kreisler, Alek, Dipl.-Chem. et al,
Deichmannhaus am Hauptbahnhof
D-5000 Köln 1(DE)

(54) **Production of cytidine derivatives.**

(57) A cytidine derivative of the formula:

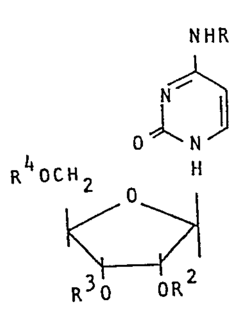

wherein $R^1$ is a carboxylic acid-derived acyl having not less than 3 carbon atoms, and $R^2$, $R^3$ and $R^4$ each represents a carboxylic acid-derived acyl, is effectively produced by subjecting a compound of the formula:

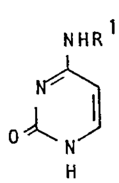

wherein $R^1$ is as defined as above and a compound of the formula:

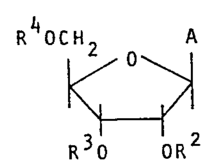

wherein A is a group of the formula $-OR^5$ where $R^5$ is a carboxylic acid-derived acyl, or a halogen, and each of $R^2$, $R^3$ and $R^4$ is as defined as above, to condensation in the presence of a Lewis acid.

EP 0 136 693 A2

# Production of Cytidine Derivatives

The present invention relates to a novel method of producing cytidine derivatives which are useful for example as an intermediate for the synthesis of pharmaceuticals.

Cytidine is an important compound as a starting material for the synthesis of cytidine diphosphate choline (the general name "Cyticholine") being currently used as a pharmaceutical.

Hitherto, there have been known a number of processes for synthesizing pyrimidine nucleosides such as cytidine through the condensation reaction between pyrimidine and sugar, and the process most customarily employed involves the condensation of silylated pyrimidine with a sugar protected with a suitable protective group [J. Org. Chem. 39, 3672-3674 (1974)]. However, the said process suffers the disadvantages that the silyl group in the silylated pyrimidine is readily hydrolyzable with water and susceptible to the attack by moisture, and also that when an acetyl group is used as a protective group for sugar, the resulting 2',3',5'-tri-O-acetylcytidine exhibits solubility in water, resulting in a lowered rate of extraction into the organic phase in the post-treatment subsequent to the reaction, and has been far from being satisfactory.

The present invention overcomes the above-described disadvantages pertaining to the conventional art, and provides an industrially favored process for producing cytidine derivatives. More particularly, the present

invention provides a method of producing a compound of the formula:

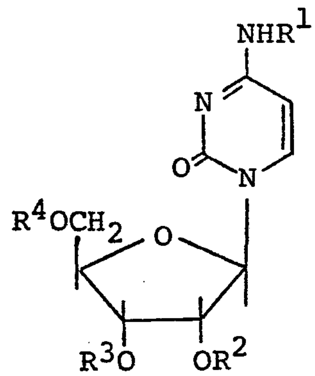

(I)

wherein $R^1$ is a carboxylic acid-derived acyl having not less than 3 carbon atoms, and $R^2$, $R^3$ and $R^4$ each represents a carboxylic acid-derived acyl, which comprises subjecting a compound of the formula:

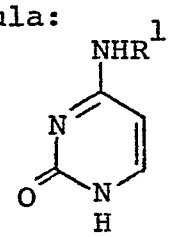

(II)

wherein $R^1$ is as defined as above and a compound of the formula:

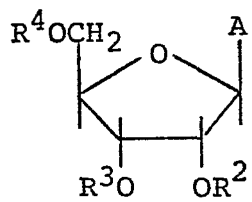

(III)

wherein A is a group of the formula $-OR^5$ where $R^5$ is a carboxylic acid-derived acyl, or a halogen, and each of $R^2$, $R^3$ and $R^4$ is as defined as above to condensation in the presence of a Lewis acid.

The carboxylic acid-derived acyl having not less than 3 carbon atoms as represented by $R^1$ in the above formulae (I) and (II) may be any of aliphatic or aromatic and straight-chain or branched-chain acy groups, and their examples include propionyl, butyryl, isobutyryl, hexanoyl, 2-ethylhexanoyl, octanoyl, decanoyl, palmitoyl and benzoyl,

with alkanoyls of 3 to 16 carbon atoms, particularly 4 to 8 carbon atoms, and benzoyl being preferred.

The halogen as represented by A in the above formula (III) may be any of fluorine, chlorine, bromine, iodine, etc., with chlorine and bromine being particularly preferred. Preferable examples of the carboxylic acid-derived acyl as represented by $R^2$, $R^3$ $R^4$ and $R^5$ include alkanoyls having a number of carbon atoms in the range of 2 to 6 and benzoyl, with the alkanoyls, especially acetyl, being particularly preferred.

The condensation reaction of the compound (II) with the compound (III) is carried out in a solvent in the presence of a Lewis acid. Normally, it is advantageous to use 1 to 1.2 moles of (III) per mole of (II). Preferred examples of the Lewis acid include metal halides such as stannic chloride, titanium tetrachloride, antimony pentachloride, aluminum chloride, ferric chloride, zinc chloride and zirconium tetrachloride, and sulfonic acid derivatives such as p-toluenesulfonic acid, trifluoromethanesulfonic acid and trimethylsilyl trifluoromethanesulfonate. Preferred examples of the solvent include halogenated hydrocarbons such as dichloromethane, 1,2-dichloroethane, chloroform, 1,2-dichloropropane and 1,1,2,2-tetrachloroethane. The reaction conditions are preferably room temperature to 100°C/1 to 30 hours.

The cytidine derivative (I) produced by the above procedure can be isolated from the reaction solution by conventional means such as solvent extraction and chromatography.

The cytidine derivatives (I) obtained by the above procedure, except the compound of the formula (I) where $R^1$, $R^2$, $R^3$ and $R^4$ are benzoyl simultaneously, all are novel compounds which have not been described in the literature. Since these novel compounds are water-insoluble and consequently can be extracted quantitatively with an organic solvent, they have the characteristic feature of being

exceptionally easily purified.

The cytidine derivatives (I) obtained by the process of the present invention can be derived into cytidine by the known alkali hydrolysis method [please refer for example to "Journal of the Americal chemical Society", 79, 5060 (1957)] or analogous methods. In such a case, the said compounds (I) are not required to be purified and are advantageously subjected as crude product to the reaction.

The compound (II), which is used as a starting material in the process of the present invention, can be produced for example by the method described in "Journal of the Chemical Society", 1956, 2384 or analogous methods.

The compound (III) can be produced easily for example by the method described in "Nucelic Acids Research", 3, 1387-1399 (1976), the method described in "Journal of the American Chemical Society", 79, 5060 (1957), the method described in "Chemistry and Industry", 1968, 547 or methods analogous to them.

According to the present invention, by subjecting the compound (II) being stable to moisture and easy to handle and the compound (III) to condensation in the presence of a Lewis acid, the objective cytidine derivative (I) can be obtained in increased yields.

The process of the present invention is industrially favored in terms of its elimination of the necessity to use mercury salts, etc.

Even when the compound (III) where $R^2$, $R^3$ and $R^4$ are acetyl simultaneously is used, furthermore, the process of the present invention offers the advantage that the resulting objective compound (I), because of its being insoluble in water, can be extracted quantitatively with an organic solvent and consequently purified exceptionally easily.

The examples and reference examples are described below to illustrate the present invention more specifically, but it is to be understood that these will not limit the scope of the present invention.

Example 1

In 30 ml of 1,2-dichloroethane were suspended 516 mg of $N^4$-benzoylcytosine and 1 g of 1-O-acetyl-2,3,5-tri-O-benzoyl-β-D-ribofuranose, and 0.4 ml of stannic chloride was added to the suspension, followed by stirring at room temperature for 15 hours. 20 ml of chloroform and 50 ml of 10% aqueous sodium hydrogencarbonate solution were added to the reaction solution, and the mixture was shaken. The precipitate, which separated out, was filtered out, and the chloroform layer was washed with water and concentrated to dryness under reduced pressure. The residue was chromatographed on 40 g of silica gel, and elution was performed with chloroform. The main fraction was concentrated to dryness under reduced pressure, and the residue was recrystallized from ethyl acetate-ethanol (6:4) to give 0.75 g of colorless needles of $N^4$,2',3',5'-tetrabenzoylcytidine, m.p. 210-212°C.

Elemental analysis, for $C_{37}H_{29}N_3O_9$

| | C(%) | H(%) | N(%) |
|---|---|---|---|
| Calcd. | 67.37 | 4.43 | 6.37 |
| Found | 67.46 | 4.55 | 6.25 |

Example 2

In 30 ml of 1,2-dichloroethane were suspended 450 mg of $N^4$-butyrylcytosine and 1 g of 1-O-acetyl-2,3,5-tri-O-benzoyl-β-D-ribofuranose, and 0.28 ml of stannic chloride was added to the suspension, followed by stirring at room temperature for 24 hours. The reaction solution was treated in accordance with Example 1, and the resulting residue was recrystallized from ethanol to give 960 mg of colorless needles of $N^4$-butyryl-2',3',5'-tri-O-benzoylcytidine, m.p. 170-173°C.

Elemental analysis, for $C_{34}H_{31}N_3O_9$

| | C(%) | H(%) | N(%) |
|---|---|---|---|
| Calcd. | 65.27 | 4.99 | 6.72 |
| Found | 65.09 | 4.69 | 6.63 |

Example 3

In 30 ml of 1,2-dichloroethane were suspended 450 mg of $N^4$-isobutyrylcytosine and 1 g of 1-O-acetyl-2,3,5-tri-O-benzoyl-β-D-ribofuranose, and 0.28 ml of stannic chloride was added to the suspension, followed by stirring at room temperature for 24 hours. The reaction solution was treated in accordance with Example 2 to give 790 mg of colorless needles of $N^4$-isobutyryl-2',3',5'-tri-O-benzoyl-cytidine, m.p. 181-183°C.

Elemental analysis, for $C_{34}H_{31}N_3O_9$

| | C(%) | H(%) | N(%) |
|---|---|---|---|
| Calcd. | 65.27 | 4.99 | 6.72 |
| Found | 65.02 | 4.86 | 6.65 |

Example 4

In 200 ml of 1,2-dichloroethane were suspended 2.84 g of $N^4$-octanoylcytosine and 3.18 g of 1,2,3,5-tetra-O-acetyl-β-D-ribofuranose, and 1.4 ml of stannic chloride was added to the suspension, followed by stirring at room temperature. The reaction solution was treated in accordance with Example 2 to give 4.4 g of colorless needles of $N^4$-octanoyl-2',3',5'-tri-O-acetylcytidine, m.p. 139-140°C.

Elemental analysis, for $C_{23}H_{33}N_3O_9$

| | C(%) | H(%) | N(%) |
|---|---|---|---|
| Calcd. | 55.75 | 6.71 | 8.48 |
| Found | 55.82 | 6.73 | 8.41 |

Example 5

In 30 ml of 1,2-dichloropropane were dissolved 462 mg of $N^4$-hexanoylcytosine and 1 g of 1-O-acetyl-2,3,5-tri-O-benzoyl-β-D-ribofuranose, and 0.4 ml of antimony pentachloride was added to the solution, followed by stirring at room temperature for 20 hours. The reaction solution was treated in accordance with Example 1 to give 850 mg of white powder of $N^4$-hexanoyl-2',3',5'-tri-O-benzoylcytidine.

Elemental analysis, for $C_{36}H_{35}N_3O_9$

| | C(%) | H(%) | N(%) |
|---|---|---|---|
| Calcd. | 66.15 | 5.41 | 6.43 |
| Found | 66.48 | 5.56 | 6.27 |

Example 6

In 30 ml of methylene chloride were dissolved 770 mg of $N^4$-palmitoylcytosine and 1 g of 1-O-acetyl-2,3,5-tri-O-benzoyl-β-D-ribofuranose, and 0.3 ml of stannic chloride was added to the solution, followed by refluxing for 6 hours. The reaction solution was treated in accordance with Example 1 to give 480 mg of a colorless oily material of $N^4$-palmitoyl-2',3',5'-tri-O-benzoylcyditine.

Elemental analysis, for $C_{46}H_{55}N_3O_9$

| | C(%) | H(%) | N(%) |
|---|---|---|---|
| Calcd. | 69.59 | 6.98 | 5.29 |
| Found | 69.28 | 6.75 | 5.01 |

Example 7

In 30 ml of chlorobenzene were suspended 400 mg of $N^4$-propionylcytosine and 1 g of 1-O-acetyl-2,3,5-tri-O-benzoyl-β-D-ribofuranose, and 490 mg of trimethylsilyl trifluoromethanesulfonate was added to the suspension, followed by stirring at room temperature for 20 hours. The reaction solution was treated with Example 1 to give 320 mg of white powder of $N^4$-propionyl-2',3',5'-tri-O-benzoylcytidine.

Elemental analysis, for $C_{33}H_{29}N_3O_9$

| | C(%) | H(%) | N(%) |
|---|---|---|---|
| Calcd. | 64.81 | 4.78 | 6.87 |
| Found | 64.58 | 4.51 | 6.65 |

Example 8

In 30 ml of 1,2-dichloroethane were suspended 636 mg of $N^4$-decanoylcytosine and 1 g of 1-O-acetyl-2,3,5-tri-O-benzoyl-β-D-ribofuranose, and 0.3 ml of stannic chloride

was added to the suspension, followed by stirring at room temperature for 20 hours. The reaction solution was treated in accordance with Example 1 to give 0.7 g of a colorless oily substance of $N^4$-decanoyl-2',3',5'-tri-O-benzoylcytidine.

Elemental analysis, for $C_{40}H_{43}N_3O_9$

| | C(%) | H(%) | N(%) |
|---|---|---|---|
| Calcd. | 66.75 | 6.02 | 5.84 |
| Found | 66.56 | 5.91 | 5.65 |

Example 9

In 30 ml of acetic acid was suspended 5.66 g of dried guanosine, and 25 g of acetyl bromide was added to the suspension, followed by stirring at room temperature for 16 hours. The precipitate was filtered out, and the filtrate was concentrated to dryness under reduced pressure over a bath at not higher than 50°C. The residue was dissolved in 50 ml of toluene, and the solution was concentrated to dryness under reduced pressure to give 2,3,5-tri-O-acetylribofuranosyl bromide as a brownish jelly-like substance. This product was dissolved in 200 ml of 1,2-dichloroethane, and 2.9 g of $N^4$-butyrylcytosine and then 2 ml of stannic chloride were added to the solution, followed by stirring at 50°C for 3 hours. The reaction solution was washed with three 200 ml portions of ice-cold water, and the organic layer was concentrated to dryness under reduced pressure to give $N^4$-butyryl-2',3',5'-tri-O-acetylcytidine as a brownish jelly-like substance. Yield of 10 g.

This product was dissolved in 50 ml of methanol, and 10 ml of an aqueous solution containing 3.3 g of sodium hydroxide was added to the solution, followed by stirring at 50°C for 2 hours. The reaction solution was concentrated to dryness under reduced pressure, and 100 ml of 1N hydrochloric acid was added to the residue, followed by adsorption on a column of 60 g of activated carbon for

chromatographic use. After the column was washed with water, elution was effected with 50% ethanol containing 2% of aqueous concentrated ammonia, and the eluate was concentrated to dryness under reduced pressure. The residue was dissolved in water, and the solution was passed through a column of 10 ml of Dowex-1 (trademark, produced by Dow Chemical Co. of U.S.A., OH type), followed by washing with water. The effluent and washing were combined and concentrated to dryness under reduced pressure, and the residue, upon addition of 20 ml of methanol, dissolved, whereupon there separated out 2.4 g of colorless needles of cytidine, m.p. 219-220°C.

Elemental analysis, for $C_9H_{13}N_3O_5$

| | C(%) | H(%) | N(%) |
|---|---|---|---|
| Calcd. | 44.44 | 5.39 | 17.28 |
| Found | 44.58 | 5.41 | 17.05 |

Example 10

In 25 ml of 1,2-dichloroethane were dissolved 2,3,5-tri-O-acetylribofuranosyl bromide prepared from 710 mg of guanosine in accordance with Example 9 and 362 mg of $N^4$-isobutyrylcytosine, and 0.3 ml of stannic chloride was added to the solution, followed by stirring at 50°C for 3 hours. The reaction solution was washed with three 25-ml portions of ice-cold water, and the organic layer was concentrated to dryness under reduced pressure. The residue was adsorbed on a column of 20 g of silica gel, and elution was effected with chloroform containing 1% of methanol. The main fraction was concentrated to dryness under reduced pressure to give $N^4$-isobutyryl-2',3',5'-tri-O-acetylcytidine as a colorless jelly-like substance. Yield of 640 mg.

Elemental analysis, for $C_{19}H_{25}N_3O_9$

| | C(%) | H(%) | N(%) |
|---|---|---|---|
| Calcd. | 51.93 | 5.73 | 9.56 |
| Found | 51.57 | 5.46 | 9.23 |

Example 11

2,3,5-Tri-O-acetylribofuranosyl bromide prepared from 710 mg of guanosine in accordance with Example 9, along with 474 mg of $N^4$-octanoylcytosine and 0.2 ml of stannic chloride, was stirred in 25 ml of 1,2-dichloroethane at room temperature for 24 fours. The reaction solution was treated in accordance with Example 10, and the resulting material was recrystallized from 5 ml of ethanol to give 700 mg of colorless needles of $N^4$-octanoyl-2',3',5'-tri-O-acetylcytidine, m.p. 139-140°C.

Elemental analysis, for $C_{23}H_{33}N_3O_9$

| | C(%) | H(%) | N(%) |
|---|---|---|---|
| Calcd. | 55.75 | 6.71 | 8.48 |
| Found | 55.89 | 6.87 | 8.19 |

Example 12

In 25 ml of 1,2-dichloroethane were dissolved 2,3,5-tri-O-acetylribofuranosyl bromide prepared from 710 mg of guanosine in accordance with Example 9 and 474 mg of $N^4$-(2-ethylhexanoyl)cytosine, and 0.2 ml of stannic chloride was added to the solution, followed by stirring at 50°C for 3 hours. 25 ml of ice-cold water was added to the reaction solution, and the mixture was shaken. The gel-like precipitate which separated out was filtered out with use of Celite. The organic layer was washed with water and then concentrated to dryness under reduced pressure, and the residue was adsorbed on a column of 20 g of silica gel, followed by elution with chloroform containing 1% of methanol. The main fraction was concentrated to dryness under reduced pressure to give $N^4$-(2-ethylhexanoyl)-2',3',5'-tri-O-acetylcytidine as a colorless jelly-like substance. Yield of 540 mg.

Elemental analysis, for $C_{23}H_{33}N_3O_9$

| | C(%) | H(%) | N(%) |
|---|---|---|---|
| Calcd. | 55.75 | 6.71 | 8.48 |
| Found | 55.88 | 6.89 | 8.26 |

Example 13

2,3,5-Tri-O-acetylribofuranosyl bromide prepared from 710 mg of guanosine in accordance with Example 9, along with 700 mg of $N^4$-palmitoylcytosine and 0.2 ml of antimony pentachloride, was boiled in 40 ml of dichloromethane for 6 hours. The reaction solution was treated in accordance with Example 12 to give $N^4$-palmitoyl-2',3',5'-tri-O-acetylcytidine as a colorless jelly-like substance. Yield of 450 mg.

Elemental analysis, for $C_{31}H_{49}N_3O_9$

| | C(%) | H(%) | N(%) |
|---|---|---|---|
| Calcd. | 61.26 | 8.13 | 6.91 |
| Found | 61.03 | 8.34 | 6.67 |

Example 14

2,3,5-Tri-O-acetylribofuranosyl bromide prepared from 710 mg of guanosine in accordance with Example 9, along with 430 mg of $N^4$-benzoylcytosine and 0.45 ml of trimethylsilyl trifluoromethanesulfonate, was stirred in 25 ml of 1,2-dichloropropane at 50°C for 5 hours. The reaction solution was treated in accordance with Example 10 to give $N^4$-benzoyl-2',3',5'-tri-O-acetylcytidine as a colorless jelly-like substance. Yield of 560 mg.

Elemental analysis, for $C_{22}H_{23}N_3O_9$

| | C(%) | H(%) | N(%) |
|---|---|---|---|
| Calcd. | 55.81 | 4.90 | 8.88 |
| Found | 55.57 | 4.62 | 8.62 |

Example 15

A 30 ml portion of diethyl ether was saturated with hydrogen chloride at 0°C, and 2 g of 1-O-acetyl-2,3,5-tri-O-benzoyl-β-D-ribofuranose was dissolved in the hydrogenchloride saturated diethyl ether, followed by allowing the solution to stand in a refrigerator for 4 days. The reaction solution was concentrated to dryness under reduced pressure, and the residue was dissolved in 20 ml of toluene.

The solution was concentrated to dryness under reduced pressure over a bath at not higher than 50°C, to give 2,3,5-tri-O-benzoylribofuranosyl chloride as a colorless jelly-like substance. This product was dissolved in 50 ml of 1,2-dichloroethane, and 500 mg of $N^4$-propionylcytosine and 0.5 ml of stannic chloride were added to the solution, followed by stirring at 50°C for 5 hours. The reaction solution was washed with three 100 ml portions of ice-cold water, and the organic layer was concentrated to dryness under reduced pressure. The residue was adsorbed on a column of 30 g of silica gel, and elution was effected with chloroform. The main fraction was concentrated to dryness under reduced pressure to give 450 mg of white powder of $N^4$-propionyl-2',3',5'-tri-O-benzoylcytidine.

Elemental analysis, for $C_{33}H_{29}N_3O_9$

| | C(%) | H(%) | N(%) |
|---|---|---|---|
| Calcd. | 64.81 | 4.78 | 6.87 |
| Found | 64.99 | 4.95 | 6.59 |

## Reference Example 1

A 2 g quantity of cytosine was refluxed in a mixed solution of 100 ml of pyridine and 4.69 g of propionic anhydride for 14 hours. After the reaction solution was cooled, the precipitate was recovered by filtration, washed with methanol and dried to give 2.84 g of white powder of $N^4$-propionylcytosine, m.p. 328-338°C (decomp.).

Elemental analysis, for $C_7H_9N_3O_2$

| | C(%) | H(%) | N(%) |
|---|---|---|---|
| Calcd. | 50.30 | 5.43 | 25.14 |
| Found | 50.42 | 5.48 | 25.03 |

## Reference Example 2

A 2 g quantity of cytosine was refluxed in a mixed solution of 100 ml of pyridine and 5.7 g of butyric anhydride for 19 hours. After the reaction solution was cooled, the precipitate was recovered by filtration, and

washed with methanol to give 2.8 g of white powder of $N^4$-butyrylcytosine, m.p. 329-334°C.

Elemental analysis, for $C_8H_{11}N_3O_2$

| | C(%) | H(%) | N(%) |
|---|---|---|---|
| Calcd. | 53.03 | 6.12 | 23.19 |
| Found | 52.97 | 6.01 | 23.19 |

Reference Example 3

A 2 g quantity of cytosine was refluxed in a mixed solution of 100 ml of pyridine and 5.7 g of isobutyric anhydride for 2 hours. The reaction solution was concentrated under reduced pressure, whereby there separated out 3.2 g of colorless plates of $N^4$-isobutyrylcytosine, m.p. 315-320°C (decomp.).

Elemental analysis, for $C_8H_{11}N_3O_2$

| | C(%) | N(%) | H(%) |
|---|---|---|---|
| Calcd. | 53.03 | 6.12 | 23.19 |
| Found | 53.25 | 6.22 | 23.19 |

Reference Example 4

A 3g quantity of cytosine was refluxed in a mixed solution of 150 ml of pyridine and 11.6 g of caproic anhydride for 24 hours. After the reaction solution was cooled, the precipitate was recovered by filtration and washed with methanol to give 5.3 g of white powder of $N^4$-hexanoylcytosine, m.p. 320-328°C (decomp.).

Elemental analysis, for $C_{10}H_{15}N_3O_2$

| | C(%) | H(%) | N(%) |
|---|---|---|---|
| Calcd. | 57.40 | 7.23 | 20.08 |
| Found | 57.38 | 7.24 | 19.82 |

Reference Example 5

A 2 g quantity of cytosine was refluxed in a mixed solution of 100 ml of pyridine and 11.8 g of capric anhydride for 22 hours. After the reaction solution was cooled, the precipitate was recovered by filtration and

washed with methanol to give 4.6 g of white powder of $N^4$-decanoylcytosine, m.p. 295-315°C (decomp.).

Elemental analysis, for $C_{14}H_{23}N_3O_2$

| | C(%) | H(%) | N(%) |
|---|---|---|---|
| Calcd. | 63.37 | 8.74 | 15.84 |
| Found | 63.63 | 8.80 | 15.36 |

Reference Example 6

A 5.55 g quantity of cytosine was refluxed in a mixed solution of 250 ml of pyridine and 41.25 g of palmitoyl chloride for 1 hour. After the reaction solution was cooled, the precipitate was recovered by filtration and washed with methanol to give 17.5 g of yellowish powder of $N^4$-palmitoylcytosine, m.p. 288-291°C (decomp.).

Elemental analysis, for $C_{20}H_{35}N_3O_2$

| | C(%) | H(%) | N(%) |
|---|---|---|---|
| Calcd. | 68.73 | 10.09 | 12.02 |
| Found | 69.22 | 10.18 | 11.02 |

Reference Example 7

A 2.22 g quantity of cytosine was refluxed in a mixed solution of 100 ml of pyridine and 5 g of caprylic acid for 2 hours. After the reaction solution was cooled, the precipitate was recovered by filtration and washed with methanol to give 3.56 g of white powder of $N^4$-octanoylcytosine, m.p. 295-300°C (decomp.).

Elemental analysis, for $C_{12}H_{19}N_3O_2$

| | C(%) | H(%) | N(%) |
|---|---|---|---|
| Calcd. | 60.74 | 8.07 | 17.71 |
| Found | 60.73 | 7.99 | 17.64 |

Reference Example 8

A 5.55 g quantity of cytosine and 10 ml of 2-ethylhexanoyl chloride were shaken in 250 ml of pyridine at 70°C for 1 hour. The reaction solution was concentrated under reduced pressure. The residue was washed with

water and recrystallized from 100 ml of methanol to give 8.4 g of colorless needles of $N^4$-(2-ethylhexanoyl)cytosine, m.p. 260-263°C.

Elemental analysis, for $C_{12}H_{19}N_3O_2$

| | C(%) | H(%) | N(%) |
|---|---|---|---|
| Calcd. | 60.74 | 8.07 | 17.71 |
| Found | 60.84 | 8.04 | 17.83 |

What is claimed is:

1. A method of producing a compound of the formula:

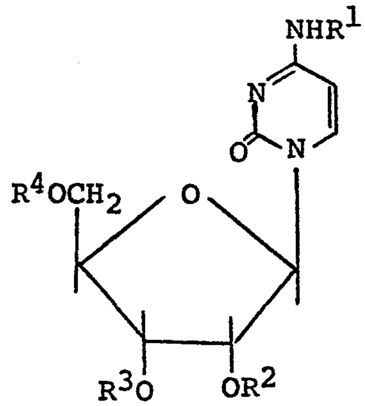

wherein $R^1$ is a carboxylic acid-derived acyl having not less than 3 carbon atoms, and $R^2$, $R^3$ and $R^4$ each represents a carboxylic acid-derived acyl,
which comprises subjecting a compound of the formula:

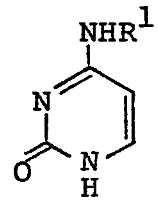

wherein $R^1$ is as defined above
and a compound of the formula:

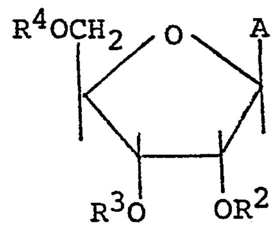

wherein A is a group of $-OR^5$ where $R^5$ is a carboxylic acid-derived acyl, or a halogen, and each of $R^2$, $R^3$ and $R^4$ is as defined above
to condensation in the presence of a Lewis acid.

2. A method of claim 1, wherein $R^1$ is alkanoyl having 3 to 16 carbon atoms or benzoyl, and $R^2$, $R^3$ and $R^4$ each

represents alkanoyl having 2 to 6 carbon atoms or benzoyl.

3. A method of claim 1, wherein A is a group of $-OR^5$ where $R^5$ is alkanoyl having 2 to 6 carbon atoms or benzoyl.

4. A method of claim 1, wherein A is bromine.

5. A method of claim 1, wherein $R^1$, $R^2$, $R^3$ and $R^4$ each is alkanoyl having 2 to 6 carbon atoms.

6. A method of claim 1, wherein the Lewis acid is a metal halide.

7. A method of claim 6, wherein the metal halide is stannic chloride.

8. A method of claim 1, wherein $R^2$, $R^3$ and $R^4$ each is acetyl and A is bromine.

9. A method of claim 1, wherein $R^1$ is butyryl.

10. A method of claim 1, wherein $R^1$ is butyryl, and $R^2$, $R^3$ and $R^4$ each is acetyl.